(19) **Europäisches Patentamt**

**European Patent Office**

**Office européen des brevets**

(11) **EP 1 733 681 A1**

(12) **EUROPEAN PATENT APPLICATION**
published in accordance with Art. 158(3) EPC

(43) Date of publication:
20.12.2006 Bulletin 2006/51

(51) Int Cl.:
*A61B 6/00* (2006.01)     *A61B 5/00* (2006.01)
*G01T 1/00* (2006.01)     *G03B 42/02* (2006.01)
*G01T 1/20* (2006.01)     *G01T 1/24* (2006.01)

(21) Application number: **05728748.4**

(22) Date of filing: **07.04.2005**

(86) International application number:
**PCT/JP2005/006852**

(87) International publication number:
**WO 2005/096944 (20.10.2005 Gazette 2005/42)**

(84) Designated Contracting States:
**DE FR GB**

(30) Priority: **07.04.2004 JP 2004113549**

(71) Applicant: **Konica Minolta Medical & Graphic, Inc.
Tokyo 163-0512 (JP)**

(72) Inventor: **TAMAKOSHI, Yasuaki
Hino-shi, Tokyo 1918511 (JP)**

(74) Representative: **Henkel, Feiler & Hänzel
Patentanwälte
Maximiliansplatz 21
80333 München (DE)**

(54) **RADIATION IMAGE CAPTURING SYSTEM AND RADIATION IMAGE CAPTURING PROGRAM**

(57) A radiography system in which an operator can confirm a radiographic image on a console currently used by the operator even when the operator moves from one console to another and radiography efficiency of the radiography can be improved, and a radiography program therefor are provided. For this sake, the radiography system includes: one or more X-ray image detectors 1 for obtaining radiographic image data and transmitting the obtained radiographic image data; and a plurality of consoles 2, each capable of obtaining an operator ID of the operator of the console 2 and, of displaying a confirmation image relating to the received radiographic image data when receiving the radiographic image data from the X-ray image detector 1. The relation between the console 2 and the operator ID of the operator currently using the console 2 is held based on the operator ID obtained by the console 2, and the confirmation image of the radiographic image can be displayed on the console 2 in which the operator ID relating to the radiographic image is the operator ID of the operator currently using the console.

*FIG.1*

EP 1 733 681 A1

**Description**

Technical Field

The present invention relates to a radiography system and a radiography program.

Background Art

Radiographic images as represented by X-ray images are widely used for medical diagnosis or the like, and a radiography apparatus is known as an apparatus for taking radiographic images. For the radiography apparatus, a type of using a radiographic film as a means for detecting radiation transmitted through a subject, and a type of using a stimulating phosphor sheet for obtaining a visible image with use of a storage phosphor (stimulating phosphor) which accumulates irradiated radiation energy.
Recently, introduction of a system having a plurality of consoles is also proposed for use in large-scale medical institutions or the like (see, for example, Patent Document 1).
Further, a radiographic image detector which employs a kind of flat surface detector referred to as an FPD (Flat Panel Detector) has recently been sold. The FPD outputs radiographic image data representative of a radiographic image detected by a plurality of solid photo-detection elements arranged two-dimensionally.
The FPD (Flat Panel Detector) is different from the radiographic film or the stimulating phosphor sheet in that it is not required to use a separate reader or the like for obtaining radiographic image data, to obtain radiographic image data just after radiography. There is a request for obtaining an image immediately after radiography , particularly on medical care sites, and therefore FPD (Flat Panel Detector) is expected to meet such a demand.
Further, large-scale medical institutions having a plurality of radiography rooms each of which has a radiation source and radiography facilities have increased. In such a case of having a plurality of rooms equipped, it is widespread to install a console for displaying radiographic images in addition to the radiation source and radiography facilities at each radiography room so as to display and confirm the radiographic image in the room immediately after radiography.
On the other hand, techniques in which a wireless communication unit and an internal power supply like a battery are arranged in a cassette with an FPD built-in and a communication with an external device, e.g., a console or the like, through wireless (i.e., radio communication), is disclosed (see, for example, Patent Documents 2 and 3).

Patent Document 1: JP 2002-159476 A
Patent Document 2: JP 2004-180931 A
Patent Document 3: JP 2004-173907 A

Disclosure of the Invention

Problems to be Solved by the Invention

In contrast with a radiographic film or a stimulating phosphor sheet, on which an image is basically taken only one time, an FPD (Flat Panel Detector) can be imaged thereon multiple times. Therefore, an operator is possible to move to another radiography room to attend next radiography with the FPD (Flat Panel Detector) carried after once imaged.
However, an obtained image could be displayed only at the console in the radiography room where the image was captured, and it was therefore inevitable for the operator to wait at the radiography room where the image was captured for confirming the captured image until the image was displayed. Accordingly, when radiography are required to carry out at a plurality of radiography rooms, the operator cannot move from one radiography room to another in a short time. As a result, it causes a problem of reducing radiography efficiency.
The present invention therefore has been made to solve the above problems. An object of the invention is to provide a radiography system in which an operator can confirm a radiographic image on a console currently used by the operator even when the operator moves from one console to another and radiography efficiency of the radiography can be improved, and to provide a radiography program for it.

Means to Solve the Problem

According to a first aspect of the invention, the radiography system includes: one or more radiographic image detectors, each detector obtaining radiographic image data representing a radiographic image captured by radiography; and a plurality of consoles, each console, when obtaining an operator ID of an operator of the console and receiving the radiographic image data representing the radiographic image from the radiographic image detector, being capable of displaying a confirmation image relating to the radiographic image with use of the received radiographic image data, wherein the relation between the console and the operator ID of the operator currently using the console is held based on the operator ID obtained by the console, and an image for confirming the radiographic image can be displayed on the console in which the operator ID relating to the radiographic image is the operator ID of the operator currently using the console.
Accordingly, even when an operator moves from one console to another to perform radiography repeatedly, the operator can confirm a confirmation image of the radiographic image data obtained by the radiography on the console at a position after movement, which is the console currently used by the operator, and therefore the operator does not need to wait for the confirmation image to be displayed on the original console to confirm the image before moving. As a result, it is possible to improve radiography efficiency of radiography.

Particularly, radiography efficiency can be improved without specific system construction by the radiographic image detector which holds the relation between the operator ID relating to the radiographic image captured by the radiography and the console.

Further, the radiography system holds the image relation among a radiographic image ID, the radiographic image detector ID and the operator ID. The radiographic image detector obtains the relation between the radiographic image ID relating to the radiographic image detector and the operator ID based on the image relation, and transmits the radiographic image data of the radiographic image to the console in which the operator ID relating to the radiographic image ID of the radiographic image is the operator ID of the operator currently using the console. Accordingly, even when the system has a plurality of radiographic image detectors, radiography efficiency of radiography can be improved without specific system construction.

Particularly, a server holds the relation between the console and the operator ID of the operator currently using the console. As a result, this permits smooth system construction, and improves radiography efficiency of radiography.

Each of the consoles holds an operator ID having the relation with the console. Accordingly, radiography efficiency of radiography can be improved without specific system construction.

In particular, the radiographic image detector holds the relation between the radiographic image ID and the operator ID, and transmits the operator ID of the radiographic image to the plurality of consoles, by using the relation. The console determines whether the received operator ID of the radiographic image matches the operator ID of the operator currently using the console, the console, when determined it matches, sends an operator ID matching signal to the radiographic image detector, the radiographic image detector, when receiving the operator ID matching signal, transmits the radiographic image data of the radiographic image to the console that sent the operator ID matching signal, and then the console can display the confirmation image relating to the radiographic image by using the received radiographic image data of the radiographic image. This allows a less amount of network communication and speedier confirmation of the image to thereby improve radiography efficiency of radiography.

A console already having the relation with one operator ID does not establish relation with other operator ID. This prevents erroneous registration of a plurality of operator IDs to one console, to allow more appropriate reception of radiographic image data and improvement of radiography efficiency of radiography.

Further, when one operator ID has newly established relation with the one console, a relation which has already been established with a console other than this console is canceled. This prevents the one operator ID to be registered on a plurality of consoles, to allow more appropriate reception of radiographic image data and improvement of radiography efficiency of radiography.

The console has an operation input unit and when a predetermined term during which the operation input unit does not have any input thereto meets a given condition, the relation between the operator and the console is canceled. Thus, when the operator does not operate the console for a long time, the relation with the operator is canceled. This allows the other operator to use the console and the console to be used efficiently, thereby to improve radiography efficiency of radiography.

Particularly, when the operator operates the console, the operator relation of the console is canceled. With this, the operation of the console by the operator allows the other operator to use the console and the console to be used efficiently, thereby to improve radiography efficiency of radiography.

At least one of the radiographic image detectors may be a portable type cassette FPD. This enhances flexibility of portability and handling, resulting in improvement of radiography efficiency of radiography.

The radiographic image detector may communicate through wireless communication. This enhances flexibility of carrying and installation with ease, thereby improving radiography efficiency of radiography.

The wireless communication may be one using a microwave or light. This allows a large capacity of radiographic image data to be transmitted at high speed efficiently, thereby improving radiography efficiency as a whole.

The system may include a wireless relay for relaying the wireless communication. According to such a structure, the detector does not wireless-communicate directly with a console that is installed in the other room in most cases, but communicates through the wireless relay, whereby a large capacity of radiographic image data can be transmitted at higher speed efficiently, to improve radiography efficiency well as a whole.

The system may include a plurality of radiography rooms, each room being provided with the radiographic image detector and the wireless relay, and the plurality of wireless relays may be connected to the plurality of consoles through a network. Accordingly, even when the radiographic image detector is moved to the other room, the detector can wireless-communicate through the wireless relay, whereby a large capacity of radiographic image data can be transmitted at higher speed efficiently, more improving radiography efficiency as a whole.

According to a second aspect of the invention, the radiography program causes a computer of a console in a radiography system, to carry out functions, wherein the system comprises:

one or more radiographic image detectors, each detector obtaining radiographic image data representing a radiographic image captured by radiography, based on an operator ID of an operator currently using the console, the ID received from the console, holding relation between the operator ID relating to the radiographic image captured by the radiography and the console, and transmitting, based on the relation, the radiographic image data of the radiographic image to the console relating to the operator ID that corresponds to the captured radiographic image; and

the plurality of consoles, each console including an operator ID obtaining unit for obtaining the operator ID of the operator, a communication unit for communicating with the radiographic image detector, a display unit for displaying a confirmation image, and the computer, and

the program causes the computer to carry out the functions as:

a relation transmission control section to control the communication unit so as to send to the radiographic image detector the operator ID of the operator currently using the console based on the operator ID obtained by the operator ID obtaining unit; and

a display control section to make the display unit display the confirmation image relating to the radiographic image by using the radiographic image data received according to the operational input to the operation input unit when the communication unit receives the radiographic image data representing the radiographic image relating to the operator ID of the operator currently using the console.

Accordingly, even when an operator moves from one console to another to carry out radiography repeatedly, the operator does not need to wait for the confirmation image to be displayed on the original console to confirm the image before moving, and can confirm the confirmation image of the radiographic image data obtained by the radiography on the console at a moving destination, the console currently used by the operator. As a result, it is possible to improve radiography efficiency of radiography.

According to a third aspect of the invention, the radiography program causes a computer of a console in a radiography system, to carry out functions, wherein the system comprises:

one or more radiographic image detectors, each detector obtaining radiographic image data representing a radiographic image captured by radiography, inquiring a server for a console to which the radiographic image data of the radiographic image are to be transmitted, and transmitting the radiographic image data of the radiographic image to the console resulted from the inquiring;

the server for communicating with one or more radiographic image detectors and a plurality of consoles, holding relation between the console and an operator ID of an operator currently using the console, and responding to the inquiry from the radiographic image detector based on the relation; and

the plurality of consoles, each console including an operation input unit to be operated by the operator, an operator ID obtaining unit for obtaining the operator ID of the operator, a communication unit for communicating with the server and one or more radiographic image detectors, a display unit for displaying a confirmation image, and a computer, and

the program causes the computer to carry out the functions as:

a relation transmission control section to control the communication unit so as to send to the server the operator ID of the operator currently using the console based on the operator ID obtained by the operator ID obtaining unit; and

a display control section to make the display unit display the confirmation image relating to the radiographic image by using the radiographic image data received according to the operational input to the operation input unit when the communication unit receives the radiographic image data representing the radiographic image relating to the operator ID of the operator currently using the console.

Accordingly, even when an operator moves from one console to another to carry out radiography repeatedly, the operator does not need to wait for the confirmation image to be displayed on the original console to confirm the image before moving, and can confirm the confirmation image of the radiographic image data obtained by the radiography on the console at a moving destination, the console currently used by the operator. As a result, it is possible to improve radiography efficiency of radiography.

According to a fourth aspect of the invention, the radiography program causes a computer of a console in a radiography system, to carry out functions, wherein the system comprises:

a plurality of radiographic image detectors, each detector obtaining radiographic image data representing a radiographic image captured by radiography, obtaining an operator ID relating to the radiographic image, transmitting the obtained operator ID relating to the radiographic image to all of a plurality of consoles, receiving an operator ID matching signal from a console, and transmitting the radiographic image data of the radiographic image to the console that sent the operator ID matching signal; and
the plurality of consoles, each console including an operation input unit to be operated by the operator, an operator ID obtaining unit for obtaining the operator ID of the operator, a communication unit for communicating with the plurality of radiographic image detectors, a display unit for displaying a confirmation image, and a computer, and
the program causes the computer to carry out the functions as:

an operator ID holding section to hold the operator ID of the operator currently using the console based on the operator ID obtained by the operator ID obtaining unit;
an operator ID matching determination section to determine whether the received operator ID matches the operator ID of the operator currently using the console when the communication unit receives the operator ID from the radiographic image detector;
an operator ID matching signal communication section to send the operator ID matching signal to the radiographic image detector when the operator ID matching determination section determines it matches; and
a display control section to make the display unit display the confirmation image relating to the radiographic image by using the radiographic image data received according to the operational input to the operation input unit.

Accordingly, the operator does not need to wait for the confirmation image to be displayed on the original console to confirm the image before moving, and can confirm the confirmation image of the radiographic image data obtained by the radiography on the console at a moving destination, the console currently used by the operator.
As a result, it is possible to improve radiography efficiency of radiography.

According to a fifth aspect of the invention, the radiography program causes a computer of a console in a radiography system, to carry out functions, wherein the system comprises:

a plurality of radiographic image detectors, each detector obtaining radiographic image data representing a radiographic image captured by radiography, obtaining an operator ID relating to the radiographic image, transmitting the radiographic image data of the captured radiographic image and the operator ID to all of a plurality of consoles; and
the plurality of consoles, each console including an operation input unit to be operated by the operator, an operator ID obtaining unit for obtaining the operator ID of the operator, a communication unit for communicating with the plurality of radiographic image detectors, a display unit for displaying a confirmation image, and a computer, and
the program causes the computer to carry out the functions as:

an operator ID holding section to hold the operator ID of the operator currently using the console based on the operator ID obtained by the operator ID obtaining unit; and
a display control section to make the display unit display the confirmation image relating to the radiographic image by using the received radiographic image data according to the operational input to the operation input unit when the communication unit receives from the radiographic image detector the radiographic image data representing the radiographic image and the operator ID, and the received operator ID matches the operator ID of the operator currently using the console.

Accordingly, the operator does not need to wait for the confirmation image to be displayed on the original console to confirm the image before moving, and can confirm the confirmation image of the radiographic image data obtained by the radiography on the console at a moving destination, the console currently used by the operator. As a result, it is possible to improve radiography efficiency of radiography.

Hereinafter, terms will be explained.
Radiation means an electromagnetic wave or a corpuscular beam that has a strong ionization action and/or a fluorescent action, including X rays, $\gamma$ rays, $\beta$ rays, $\alpha$ rays, deuteron beam, proton beam and other heavily charged particle beam and neutron beam. In the invention, it is preferable to use as radiation an electron beam, X rays and $\gamma$ rays, and particularly, X rays.
Console means an apparatus for an operator to communicate with a cassette, and may be connected with a separate display device or an operation device, or may be integrated with a display device or an operation device.
Radiographic image detector means an apparatus for obtaining radiographic image data representative of a radiographic image by radiography, including, but not limited, a cassette, apparatus for erect radiography, apparatus for supine radiography and the like. The cassette includes a case of having an internal power supply for supplying power, and a case of being supplied power from an external power supply. In the case of having an internal power supply for supplying power, the cassette may preferably have a plurality of different power supply states so as to change the power supply states at a suitable timing. Such power supply states preferably include, for example, a radiography-ready state and another state having lower power consumption than the radiography-ready state. It is particularly preferable that the detector has, as the state having lower power consumption than that of the radiography-ready state, one or more states under control of a radiography-standby mode, and a state under control of a sleep mode with much lower power consumption.

Radiography operation means an operation necessary for obtaining radiographic image data by radiography. In a panel shown in an embodiment, for example, the operation includes each operation of initialization of the panel, accumulation of electric energy generated by irradiation of radiation, reading of electric signals, and conversion to image data.

The radiography-ready state means a state in which the radiographic image data are ready to be obtained by the radiography operations.

Communication between the console and the radiographic image detector may be a wired communication using an electric wire or an optical fiber, or a wireless communication using an ultrasonic wave, a radio wave, light or the like. The wireless communication may be a direct wireless communication between the console and the radiographic image detector, or a wireless communication performed through a wireless relay device equipped on the way. The communication may be an analogue one or a digital one.

Brief Description of the Drawings

[FIG. 1] This is a view showing a schematic configuration of a radiography system according to an embodiment.

[FIG. 2] This is a view showing a structure of a bed for radiography and the surrounding in case of using a bed for radiography as the radiography system according to the embodiment.

[FIG. 3] This is a block diagram showing a configuration of main parts of a radiographic image detector according to the embodiment.

[FIG. 4] This is a block diagram showing a configuration of main parts of a console according to the embodiment.

[FIG. 5] This is a flowchart showing a flow of establishing operator relation in the console according to the embodiment.

[FIG. 6] This is a flowchart showing a flow of canceling the operator relation due to an elapse time according to the embodiment.

[FIG. 7] This is a view showing a schematic configuration of a radiography system according to a fifth embodiment of the present invention.

Preferred Embodiment of the Invention

This column "Preferred Embodiment of the Invention" is a column for specifying and explaining an embodiment contemplated to be best in the invention. Terms used in the scope of the invention or in the scope of the claims may appear to be conclusive or definitive expressions, but these are expressions to specify the embodiment contemplated best by the inventors, and not to specify or limit the terms used in the scope of the invention or in the scope of the claims.

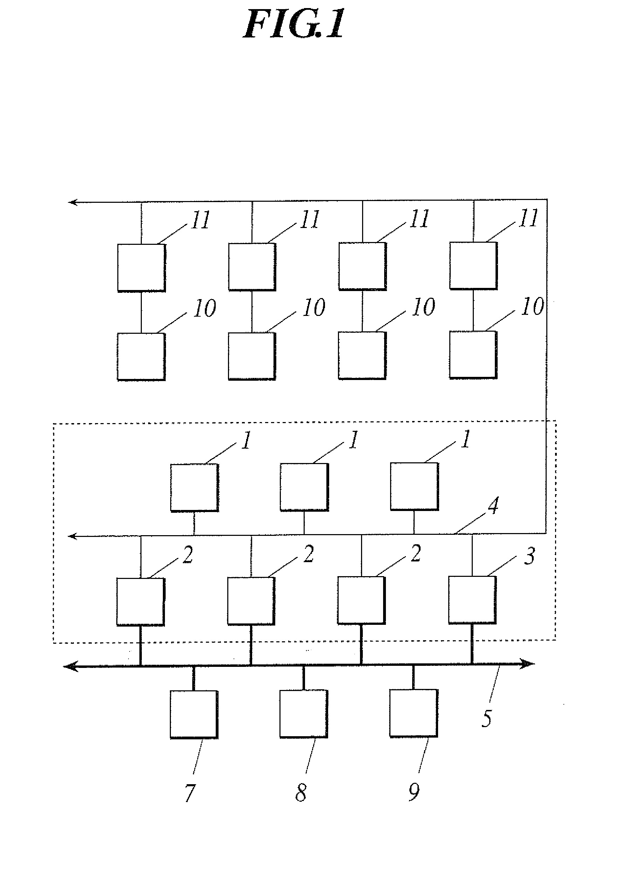

Hereinafter, one embodiment of a radiography system and radiography program will be explained with reference to FIGS. 1 to 6.

<First Embodiment>

In a radiography system of the embodiment, a plurality of X-ray image detectors 1, a plurality of consoles 2 and a server 3 are connected to each other through a network 4. X-ray irradiation controllers 11 connected with respective X-ray sources 10 for radiating X rays are also connected to the network 4. Each of the plurality of X-ray image detectors 1 corresponds to a radiographic image detector of the invention, which reads the intensity of radiation by solid photo-detection elements arranged two-dimensionally and outputs X-ray image data representative of an X-ray image. Each of the plurality of consoles 2 sends detecting conditions to the X-ray image detectors 1. Each of the plurality of consoles 2 receives from the X-ray irradiation controller 11 a radiography start signal indicating X-ray irradiation start timing, and transfer it to the X-ray image detector 1. Each of the plurality of consoles 2 receives X-ray image data transmitted from the X-ray image detector 1. Each of the plurality of consoles 2 displays a confirmation image relating to the radiographic image using the received radiographic image data. Each of the plurality of consoles 2 performs image processing for the received radiographic image data. The server 3 manages patient information relating to a patient 6 as a subject, radiography information relating to the radiography including radiography conditions, detector IDs proper to respective X-ray image detectors 1, and detector information including radiography conditions and image processing conditions.

The X-ray irradiation controller 11 controls X-ray irradiation by the X-ray source 10 according to the operation by an operator. That is, the X-ray irradiation controller 11 sets X-ray irradiation conditions for the X-ray source 10 depending on the operation by the operator, and transmits to the X-ray source 10 and the console 2 the radiography start signal indicative of the X-ray irradiation start timing depending on operation of operation buttons, not shown.

The X-ray source 10 radiates X rays when receiving the radiography start signal. Here, the X-ray source 10 is connected with an X-ray power supply, not shown. The X-ray power supply supplies to the X-ray source 10 the power necessary for radiating X rays. When receiving the radiography start signal, each of the plurality of consoles 2 sends the radiography start signal to the X-ray image detector 1 relating to the X-ray source 10 controlled by the X-ray irradiation controller 11 that has sent the radiography start signal. The X-ray image detector 1, when receiving the radiography start signal, carries out the operation relating to the X-ray irradiation.

The X-ray source 10 usually receives a radiation-preparing signal from the X-ray irradiation controller 11 to prepare radiation, then, receives the radiography start signal to generate X rays. The X-ray irradiation controller 11 may send the radiation-preparing signal to the console 2. When receiving the radiation-preparing signal, each of the plurality of consoles 2 sends the radiation-preparing signal to the X-ray image detector 1 relating to the X-ray source 10 controlled by the X-ray source controller that has sent the radiation-preparing signal. The X-ray image detector 1 prepares for the X-ray irradiation when receiving the radiation-preparing signal.

Each of the plurality of consoles 2 is connected with a DICOM network 5. The DICOM network 5 can be connected with an image output device 7 such as a laser imager, an image diagnostic apparatus 8 having a medical-image diagnosis monitor such as a CRT monitor and FPD (Flat Panel Detector) monitor, and an image filing device 9 for electronically storing radiographic image data so that rewriting is impossible. The image output device 7 outputs onto a film the radiographic image data that the console 2 outputs to provide a doctor with a visualized diagnostic image. The image diagnostic apparatus 8 displays on the monitor the radiographic image data that the console 2 outputs to provide a doctor with a visualized diagnostic image. The image-filing device 9 stores the radiographic image data that the console 2 outputs. The radiographic image data stored in the image-filing device 9 can be output to the image output device 7 and the image diagnostic apparatus 8, when required. The network 4 may be a communication line dedicated to the system, and may be preferably an existing line such as Ethernet (TM), because the dedicated line reduces flexibility of the system.

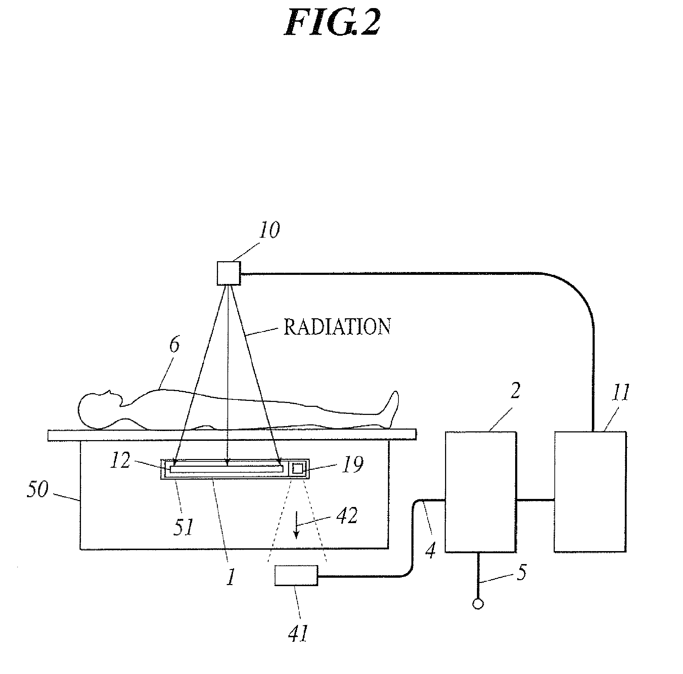

FIG. 2 shows a bed for radiography 50 and its surroundings as an embodiment of the radiography system. The bed 50 for radiography is a bed on which a patient 6 is placed as a subject for X-ray radiography. The X-ray source 10 is provided over the bed for radiography 50. The bed 50 for radiography has a cassette insertion part 51 for setting the X-ray image detector 1. The X-ray image detector 1 incorporates a flat surface detector 12, which will be explained later. When the X-ray image detector 1 is set to the cassette insertion part 51, the flat surface detector 12 is disposed within an irradiation area of the X rays radiated from the X-ray source 10. The X-ray image detector 1 has a wireless communication unit 19 embedded therein for communicating with the console or the like by wireless communication, which will be explained later. It is preferable for the wireless communication unit 19 to send with directivity. In this case, a wireless communication unit 41 of the network 4 is disposed on the directivity direction 42. The X-ray image detector 1 is connected to the network 4 through the wireless communication unit 41. FIG. 2 shows, as an example, an equipment structure of the bed 50 for radiography and its peripherals for supine radiography that images a patient lying on the bed, but it is also applicable to an equipment structure for erect radiography that images a patient in a standing state, or to a combined structure.

First, the X-ray image detector 1 of the embodiment will be explained.

The X-ray image detector 1 is a cassette type FPD (Flat Panel Detector) accommodating an FPD (Flat Panel Detector) as the flat surface detector 12. The X-ray image detector 1 in the embodiment is explained using a cassette type FPD (Flat Panel Detector) as one example, but not limited to that having an FPD (Flat Panel Detector) as the flat surface detector or to that of a cassette type.

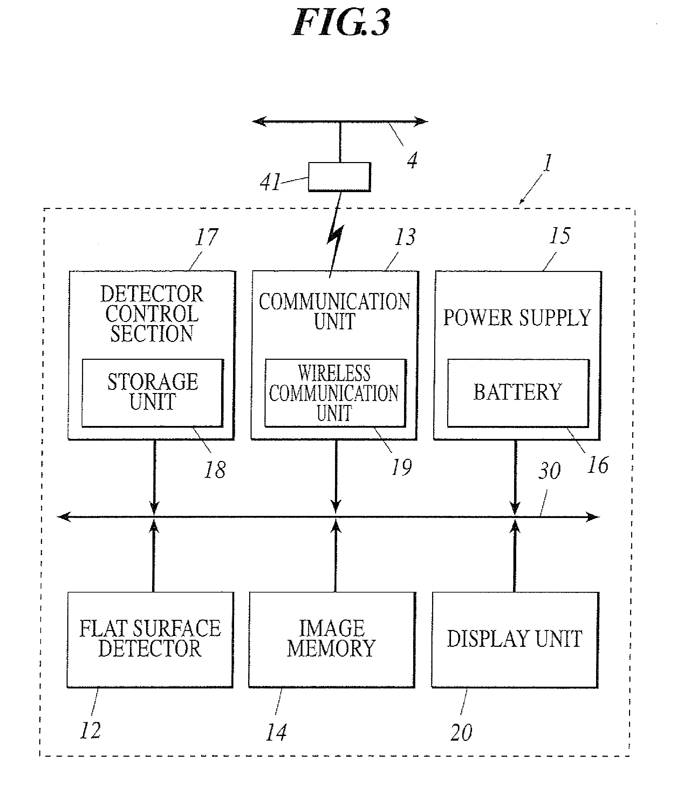

FIG. 3 is a block diagram showing a configuration of main parts of the X-ray image detector 1 of the embodiment. As shown in FIG. 3, the X-ray image detector 1 includes a flat surface detector 12. The flat surface detector is an image data capturing means having a plurality of solid photo-detecting elements arranged two-dimensionally for outputting the radiographic image data. When radiography, the X-ray image detector 1 is arranged so that the flat surface detector is disposed facing the radiography region of the patient within the irradiation field of the X-ray source 10 to detect X rays radiated from the X-ray source 10.

As for the flat surface detector 12, there may be of an indirect type and of a direct type, but not limited to these types. The indirect type detector has, on a predetermined board such as a glass board for example, an X-ray-photo conversion layer for converting X rays to fluorescence, and a photoelectric conversion layer for detecting the fluorescence converted by the X-ray-photo conversion layer to convert to electric signals. The direct type detector has an X-ray-charge conversion layer for converting X rays to electric charge directly instead of the X-ray-photo conversion layer and the photoelectric conversion layer.

Each solid photo-detecting element of the flat surface detector 12 converts X rays, radiated from the X-ray source 10 toward the patient 6, into electric charge according to the intensity of the X rays transmitted through the patient 6, accumulates the charge, which is read out by a reading signal, and outputs the radiographic image data. Thus, the radiographic image data representative of a radiographic image are obtained by X-ray radiography. This radiographic image data represents the radiographic image of intensity distribution of the X rays transmitted through the patient 6.

The X-ray image detector 1 has a communication unit 13 that can send the radiographic image data to any one of the plurality of consoles 2. The communication unit 13 includes a wireless communication unit 19 embedded therein for sending and/or receiving data by an electromagnetic wave such as a radio wave and light. In case of radio wave communication, wireless transmission by the wireless communication unit 19 may preferably have directivity to a directive direction 42 so as not to irradiate the patient 6 by a strong radio wave. The wireless communication between the wireless communication unit 19 and the wireless communication unit 41 of the network 4 enables sending and receiving of various information between the console 2 or the like. Here, one or more X-ray image detectors 1 and the plurality of consoles 2 may form a wireless LAN through the wireless communication units 19 and the wireless communication units 41.

Further, the X-ray image detector 1 includes an image memory 14 that can store radiographic image data relating to a plurality of radiographic images. The image memory 14 temporarily stores radiographic image data detected by the flat surface detector 12, and is composed of, for example, a nonvolatile memory such as flash memory or the like. Since the image memory 14 can store radiographic image data relating to the plurality of radiographic images while sending the radiographic image data to the console 2, it can store another radiographic image data due to another radiography. Further, because the detector 1 has such a large capacity of memory, it can continuously accept plural times of radiography without sending radiographic image data to the console 2 each time of radiography.

The X-ray image detector 1 also includes a power supply 15 for supplying power to the flat surface detector 12, communication unit 13, image memory 14, detector control section 17 and display unit 20. The power supply 15 has a battery 16, which can be charged through a charging terminal (not shown) provided at a given position of the X-ray image detector 1. Since the X-ray image detector 1 has the power supply 15, it can continuously output radiographic image data due to radiography and send the radiographic image data.

The X-ray image detector 1 further includes a detector control section 17 for controlling flat surface detector 12, communication unit 13, image memory 14, power supply 15 and display unit 20. The detector control section 17 is a computer and has, for example, a CPU (central processing unit) and storage 18 that stores various programs and data. The programs stored in the storage 18 enable the computer to function as the control section 17, to be described below. The control section 17 controls the communication unit 13 to transmit the radiographic images obtained by radiography.

Here, each of detector control section 17, flat surface detector 12, communication unit 13, image memory 14 and power supply 15 is connected with a bus 30.

For wireless communication by the wireless communication section 19 of the communication unit 13, communication by an ultrasonic wave, a radio wave or light may be used, but not limited thereto. In order to transfer large capacity of data, such as image data, it is preferable to use a microwave (i.e. ultrahigh frequency wave, centimeter wave, millimeter wave or sub-millimeter wave) as an electromagnetic wave having a frequency ranging from $3\times10^2$ MHz to $3\times10^2$ GHz, or light (that is, an electromagnetic wave having a frequency higher than terawave, that is, higher than $3\times10^2$ GHz).

Generally, as radio-wave communication, for example, a method by a specified low power radio-wave using a $7\times10$ MHz band or a $4\times10^2$ MHz band, a method by PHS, a method by a next generation portable phone using a 1.4 GHz band, 2 GHz band or 2.1 GHz band, a method by a wireless LAN using a 2.4 GHz band or 5.2 GHz band, which conforms to regulations IEEE 802.11a, 802.11b or 802.11g, a method by FWA (fixed wireless access) using a 18 GHz band or 19 GHz band, a method based on wireless communication regulations including methods by Bluetooth using a 2.45 GHz band and Home RF (home radio frequency) using a 2.4 GHz band, a communication method using an UWB (ultra wide band) radio wave, and a method by an ISM (industrial scientific and medical) band using a 2.4 GHz band or 5.8 GHz band, may be used. It is preferable to use a radio wave having a frequency more than $3\times10^2$ MHz (particularly, more than 1 GHz) from the viewpoint of high-speed communication and a small-sized antenna.

Light and a high-frequency radio wave have strong straightforwardness and directivity, and communication using such a wave causes communication failures, reduction of data-transfer speed, and un-communicable state in wireless communication in which communication cannot be performed, because of shadowing, multi-pass fading, radio-wave conditions and radio-wave interference. The shadowing occurs when an obstacle on a communication path shuts the signal and the radio wave is hard to reach behind it. The multi-pass fading occurs when various reflected waves are combined and weaken the signal. Therefore, it is preferable to use a radio wave having a frequency less than $3\times10$ GHz (particularly, less than 3 GHz), from the viewpoint of radio-wave detour with less shadowing and multi-pass fading, a small-sized communication circuit and lower cost.

The communication unit 31 may have a structure capable of relating to light or radio waves having a plurality of frequencies, by having a plurality of wireless carriers and antennas (none of these shown) or light receivers and generators for sending and receiving electromagnetic waves with different frequencies or light. In this case, communication may be performed using a wireless carrier capable of sending and receiving an electromagnetic wave with less than 3 GHz (particularly 1 GHz) for various signals necessary for secure communication, such as a signal notifying X-ray irradiation timing and an instruction signal to instruct reading timing of image data, and using a wireless carrier capable of sending and receiving a microwave with a higher frequency described above or light for large capacity of data like image data. Thus, communication may be performed differently according to the kinds of signals. The communication unit 31 may have a structure capable of switching frequencies of electromagnetic waves to be sent and received so that communication can be performed by appropriately changing the frequency according to the kinds of signals to be sent and received.

Here, the wireless communication described above may be analogue one or digital one.

Next, a console 2 applied to the embodiment will be explained.

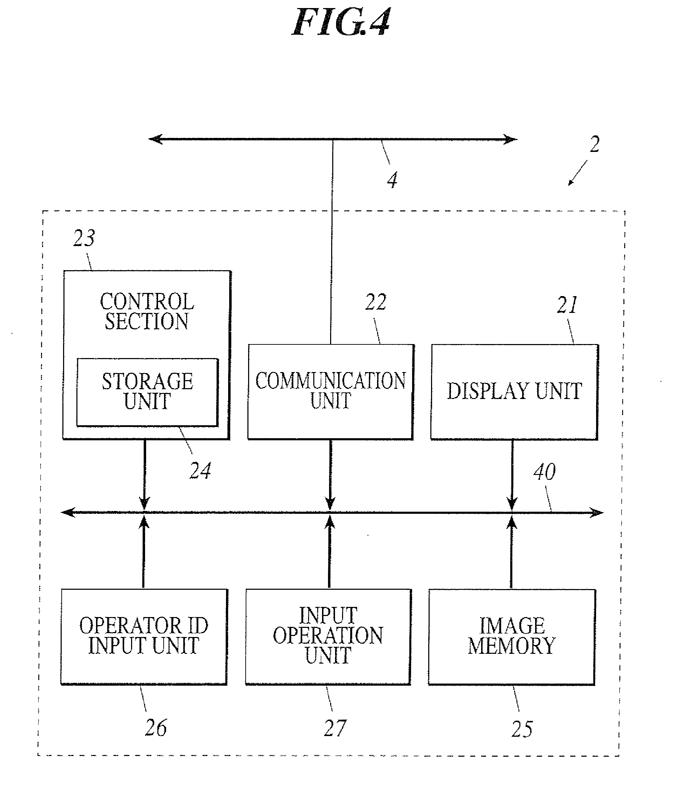

FIG. 4 is a block diagram showing a configuration of main parts of the console according to the embodiment.

As shown in FIG. 4, the console 2 has an operator ID input unit 26 constituting an operator ID obtaining means for obtaining an operator ID of the console operator. In the operator ID input unit 26, the ID of an operator operating the console 2 can be input, for example, through a physical characteristic detector including a fingerprint detector or a voiceprint detector that can distinguish identification information based on a physical characteristic of the operator itself such as the fingerprint or the voiceprint, a reader capable of reading one of belongings that the operator carries, a keyboard or a touch panel. It is preferable as the operator ID input unit 26, because of low cost and easiness of obtaining accurate information, to use a reader for reading one of the belongings that the operator carries, such as an ID reader to read an ID card, a bar code reader to read a bar code label, and a portable signal receiver to receive a signal from a portable generator.

Here, without being provided with the operator ID input unit 26 separately, an operation input unit 27, to be explained later, can be configured to act also as the operator ID input unit 26, for inputting before performing input operation from the operation input unit 27. In order to save a troublesome input at every operation, once inputting the operator ID, it is convenient to set it as a default when only one person operates one console 2. Alternatively, it is also possible that IDs of operators who use the console are registered in advance so that an ID can be selected through a keyboard or a touch panel.

The console 2 has an operation input unit 27 for inputting patient information, radiography information and the like. The operation input unit 27 includes, but not limited thereto, for example, an operation panel and a mouse, a keyboard, a touch panel, a voice input device or the like, and may be configured with one or more of these devices. An operation signal from the operation panel or the mouse, a key depression signal due to depressing operation of a key on the keyboard, or the like are output to a control section 22 as an input signal.

The console 2 has an operation input unit 27 for inputting various instructions, given information and the like. The operation input unit 27 includes, for example, an operation panel and a mouse, a keyboard, a touch panel, a voice input device or the like, and outputs, to a control section 22 as an input signal, an operation signal from the operation panel or the mouse, a key depression signal due to depressing operation of a key on the keyboard, or the like. The structure of the operation input unit 27 is not limited to those described above, as long as they can set various processing contents. The instructions and the information are also not limited to those exemplified above.

From the operation input unit 27, for example, patient information of a patient to be imaged by the X-ray image detector 1 and radiography information for radiography the patient are input before X-ray radiography. Here, the patient information includes patient name, age, sex, birth date, patient ID for identifying the patient, etc. The radiography information includes information about regions to be imaged (information about what physical part of the patient is to be imaged), and about radiography methods (information for identifying radiography directions, such as posteroanterior projection, anteroposterior projection, lateral radiography, oblique radiography, and for identifying pseudo imaging technique). These information are used not only for a radiography record of a patient, but also as parameters for determining image processing conditions of read radiographic image data, particularly tone conversion processing conditions.

Meanwhile, the regions to be imaged may be selectable with two steps of rough major grouping, based on primary parts of the human body, and detailed breakdowns. As examples of the major grouping, they are, for example, head, chest, abdomen, upper limbs, lower limbs, backbone, pelvis, etc. The breakdown is what is classified to more detailed regions for a region represented by a major grouping. When the major grouping is, for example, the "upper limbs", its breakdown includes shoulder joint, shoulder blade, acromioclavicular joint, humerus, cubita joint, forearm bone, hand joint, finger bone, etc. The radiography directions are generally those against the human body, but not limited thereto. Such radiography directions are typically PA (posteroanterior projection), AP (anteroposterior projection), LAT (lateral radiography), oblique radiography, and the like.

The console 2 includes a display unit 21 for displaying not only variety of information but also a confirmation image relating to the radiographic images with use of the received radiographic image data. The display unit 21 is, for example, a CRT display or a FPD (Flat Panel Detector) monitor, and displays character information as well as radiographic image data. Contents to be displayed includes, but not limited to, numbers for identifying the X-ray image detector 1 from which the radiographic image data has been transmitted and obtained and the radiography room where the radiography has been performed, operator ID, patient information, radiography information, reading conditions, X-ray radiography conditions such as a tube voltage and a dose of the X-ray source 10, the number of pixels and matrix size of radiographic image data, the number of bits per pixel for radiographic image data, type of image processing, image processing parameters, information such as contents of correction processing, and an image of radiographic image data.

The console 2 also includes a communication unit 22 for sending and receiving information between an X-ray image detector 1 and an external device. The communication unit 22 receives radiographic image data transmitted from the X-ray image detector 1. The communication unit 22 also sends to the X-ray image detector 1 variety of information, such as the patient information registered in the console 2 in advance and the radiography information, operator ID of an operator operating each console 2, and the like.

The console 2 also includes an image memory 25 for storing the radiographic image data that the communication unit 22 has received, the radiographic image data after application of image processing to the received radiographic image data, and confirmation image data for displaying the confirmation image.

The console 2 also includes a console control section 23 for controlling each of operator ID input unit 26, operation input unit 27, display unit 21, communication unit 22 and image memory 25. The control section 23 includes a storage unit 24 for storing various programs and data and for temporarily storing an operator ID input from the operator ID input unit 26 in association with the patient information and the radiography information input from the operation input unit 27. The programs stored in the storage unit 24 make a computer to function as the control section 23, which will be described below.

Each of operator ID input unit 26, operation input unit 27, display unit 21, communication unit 22, image memory 25, and control section 23 is connected to a bus 40.

The consoles 2 and the server 3 can be connected to a hospital information system (HIS) and a radiography information system (RIS). In this case, it is preferable to obtain the patient information and the radiography information on-line through these HIS and RIS. By allowing each patient to have a portable storage medium storing these information, and the console 2 to have a storage-medium reader for reading the information stored in the portable storage medium, the information, such as the patient information and the radiography information, may be read from the portable storage medium which the patient brings with. Such a portable storage medium and a storage-medium reader respectively include, but not limited to, a bar code and a bar code reader, a magnetic card and a magnetic card reader, an IC card and an IC card reader.

It is also possible to retrieve from HIS or RIS the information that matches a patient ID. In this case, by allowing the patient to have a portable storage medium storing the patient ID and preparing a storage-medium reader, these information may be read from the portable storage medium which the patient brings with. Alternatively, the information may be input through the operation input unit 27. By storing information unique to a patient, such as a finger print or a voice print, and installing a finger print reader or a voice print reader to the console 2, it is also possible to retrieve from HIS or RIS the information that matches the finger print or the voice print.

A radiography program stored in the storage unit 24 of the control section 23 makes the control section 23 function as an operator ID holding means for holding an operator ID of the operator currently using the console based on the operator ID obtained through the operator ID input unit 26, a detector ID obtaining means for obtaining a radiographic image detector ID relevant to a radiographic image, an image relation establishing means for establishing an image relation among a radiographic image ID, the radiographic image detector ID obtained by the detector ID obtaining means and the operator ID, an image relation transmission control means for making the X-ray image detector 1 transmit to the communication unit 22 the relation between the radiographic image ID and the operator ID associated with the X-ray image detector 1 based on the image relation, an operator ID transmission control means for making the X-ray image detector 1 transmit to the communication unit 22 the operator ID of the operator currently using the console, and a display control means for displaying a confirmation image, when the communication unit 22 receives from the X-ray image detector 1 radiographic image data representing the radiographic image, relating to the radiographic image on the display unit 21 using the received radiographic image data according to the operation input to the operation input unit.

A description will now be given of a flow of the radiography program in the console 2 according to the embodiment.

When the operator ID is input from the operator ID input unit 26 of the console 2, the control section 23 functions, with the radiography program, as the operator ID holding means for holding the operator ID of the operator currently using the console 2 based on the operator ID obtained through the operator ID input unit 26.

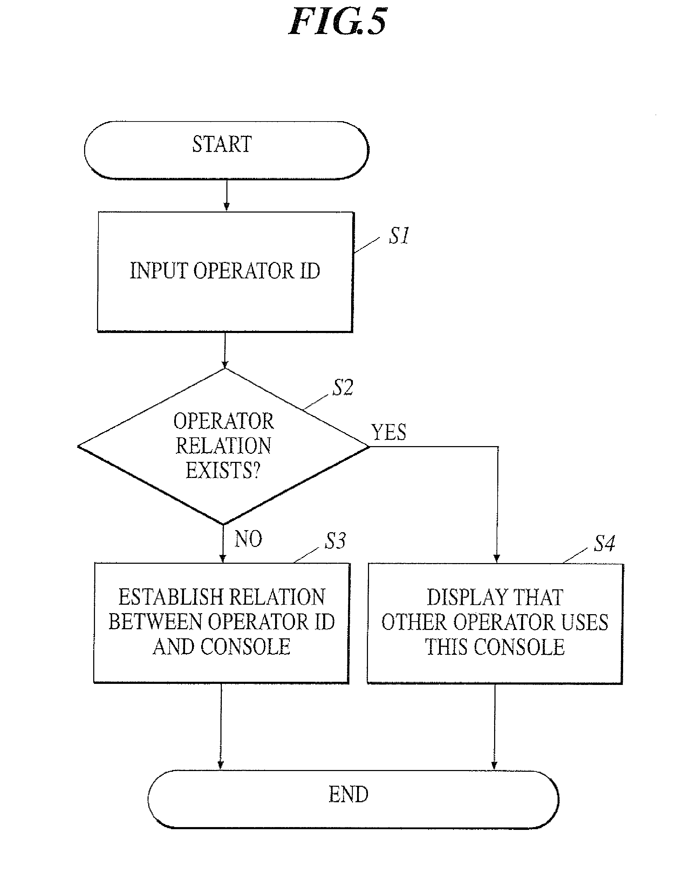

Specifically, the control section 23 executes the flow of establishing the operator relation, which is shown in FIG. 5. That is, when the operator ID is input from the operator ID input unit 26 (step S1), it is determined whether an operator ID that has been already registered and established the operator relation exists or not in the console 2 (step S2). When other operator ID that has been established the operator relation does not exist, the input operator ID is registered, the operator relation with the console 2 is established, the communication unit 22 is controlled so as to send to the other console 2 a specific operator relation canceling signal that instructs to cancel the operator relation with the input operator ID (step S3), and the flow of establishing the operator relation ends. When receiving the specific operator relation canceling signal, the other console 2 cancels the operator relation when it holds the operator relation with the operator ID. On the other hand, when other operator ID that has been established the operator relation exists, the display 21 is controlled to display that the other operator is in use of this console 2 (step S4), and the flow of establishing the operator relation ends.

When the communication unit 22 receives the specific operator relation canceling signal from the other console 2, the control section 23 determines whether the operator ID indicated by the specific operator relation canceling signal matches the operator ID now held. When determined to match, the control section 23 cancels the holding of the operator ID, that is, cancels the operator relation. When determined not to match, the control section 23 does not particularly change the state.

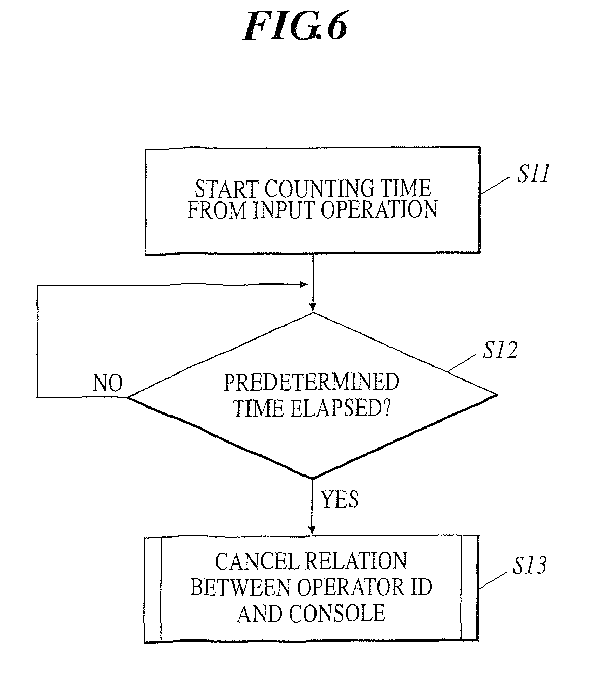

When a predetermined input is performed in the operation input unit 27, the control section 23 executes the flow of canceling the operator relation due to elapse time, which is shown in FIG. 6. That is, the control section starts counting elapse time from operation of the predetermined input through the operation input unit 27 (step S11), and determines whether a predetermined time has elapsed (step S12). When determined that the predetermined time has not elapsed, the flow returns to step S12, and when determined that the predetermined time has elapsed, the control section cancels the operator relation between the operator ID and the controller 2, stops counting the elapse time (step S13), and the flow of canceling the operator relation due to elapse time ends. The predetermined input may be all of operational inputs, or specific operational inputs, such as movement of a focus and click of a button. This may be determined by balancing achievement of targeted functions with reduction of responsiveness according to the performance of the control section 23. The predetermined time may be a constant time determined in advance, or a time variable depending on a status of the console 2 or of the entire system. Alternatively, the control section may determine not by whether the predetermined time has elapsed, but by whether the elapse time satisfies a predetermined condition. For instance, the control section may determine whether a function value falls within a predetermined range or outside the range, the function being given by the following expression:

$$\alpha 1 < F(T, U) < \alpha 2,$$

where F( ) is a function, T an elapse time, U an other element, $\alpha 1$ a lower limit value for canceling, and $\alpha 2$ an upper limit value for canceling.

The control section 23 determines whether a predetermined input operation is applied to the operation input unit 27, and when determined that the predetermined input operation is applied, the control section cancels the operator relation between the operator ID and the console 2. In this case, after application of the predetermined input operation, the control section determines whether the operator ID input to the operator ID input unit 26 matches the currently held operator ID, and when determined to match, the control section cancels the operator relation, which preferably prevents cancellation by other operators. Further, after application of the predetermined input operation, the control section determines whether the operator ID input to the operator ID input unit 26 matches the ID of an operator having the supervisor privilege on the console 2, and when determined to match, the control section cancels the operator relation, which preferably allows emergency action and preventing cancellation by other operators.

As described above, by holding the operator ID of the operator currently using the console based on the operator ID obtained from the operator ID input unit 26, the control section 23 of the console 2 holds the operator relation between the console and the operator ID of the operator currently using the console. Each of the plurality of consoles 2 holds the operator relation between the console and the operator ID, which means that the entire radiography system holds the operator relation between each console and the operator ID of the operator currently using the console.

In addition, when the control section 23 of the console 2 holds the aforementioned operator relation, operation input to the operation input unit 27 causes the control section 23 to identify an X-ray image detector 1 that outputs radiographic image data due to radiography to obtain a radiographic image detector ID. The control section 23 of the console 2 establishes an image relation associating a radiographic image ID fixedly issued automatically with the currently held operator ID and the obtained radiographic image detector ID, and stores it temporarily.

At this time, the operation input to the operation input unit 27 causes the control section 23 of the console 2 to associate the image relation with the aforementioned radiography information and the patient information relevant to the radiography, and to set detecting conditions in the X-ray image detector 1 from these kinds of information. The control section 23 controls the communication unit 22 so as to send the operator ID, the radiographic image ID and the detecting conditions to the X-ray image detector 1 relating to the radiographic image detector ID before radiography.

Simultaneously, with the setting of the aforementioned detecting conditions, based on the detector information, relevant to the X-ray image detector 1, radiography information and patient information, the control section 23 of the console 2 sets image processing conditions for the radiographic image data transmitted from the X-ray image detector 1 and X-ray source control information for controlling the X-ray source 10, and controls the communication unit 22 so as to send the X-ray source control information to the X-ray irradiation controller 11 to control the X-ray source 10 before radiography.

Meanwhile, out of the information input from the operation input unit 27, reusable information may be kept as default values to simplify inputting for the next time and after. When the radiography information and the patient information have been registered in advance, these kinds of information may be listed on the display unit 21, and the operator may select the necessary information from the displayed list.

When the communication unit 13 receives from the console 2 the operator ID, radiographic image ID and detecting conditions, the detector control section 17 of the X-ray image detector 1 stores the received operator ID, radiographic image ID and detecting conditions into the storage unit 18. The detector control section 17 sets the conditions of the flat surface detector 12 based on the received detecting conditions. When the communication unit 13 receives an X-ray irradiation signal sent from the console 2 or the X-ray irradiation controller 11, the detector control section 17, at the time of radiography, controls to output radiographic image data that the flat surface detector 12 detected based on the detecting conditions. The control section 17 temporarily stores the output radiographic image data into the image memory 14, and also stores the radiographic image ID, the operator ID and the memory addresses of the image data on the image memory 14.

When the image memory 14 stores the radiographic image data by its whole screen, the control section 17 inquires the all consoles 2 for the holding operator ID to retrieve a console 2 that holds the operator ID relating to this radiographic image data.

When the communication unit 22 of the console 2 receives the signal inquiring the operator ID from the X-ray image detector 1, the control section 23 controls to send to the inquiring X-ray image detector 1 the operator relation between the operator ID, held by the communication unit 22, and the console ID of the console.

Based on the responded result of the operator relation between the operator ID and the console ID, which is responded from each console 2 responding to the signal inquiring the operator ID, the X-ray image detector 1 determines the console 2 that holds the operator relating to the radiographic image data, and transmits the radiographic image data with the radiographic image ID to the console 2 holding the operator ID relating to the radiographic image data.

When any of the consoles 2 does not hold the operator ID relating to the radiographic image data, a display part, not shown, (for example, an LED or the like) displays that a destination to be sent is unknown. When the operator sees this display, the operator, after registering the operator ID at any one of the consoles 2, instructs from the console 2 the X-ray image detector 1 to transmit the radiographic image data. That is, when the control section 23 of the console 2 receives a resending instruction signal for instructing the specific X-ray image detector 1 to resend according to the input from the input operation unit 17 in a state in which the operator ID is held due to the input of the operator ID from the operator ID input unit 26, the detector inquires all consoles 2 for the holding operator ID, and therefore above process is restarted.

When the communication unit 22 receives the radiographic image data, the control section 23 of the console 2 executes the image processing on the received radiographic image data based on the setting as described above, and stores into the image memory 25. At this time, kinds of image processing executed by the controller include, but not limited to, tone conversion processing to convert the tone of the radiographic image data, frequency conversion processing to convert the frequency characteristic of the radiographic image data, and dynamic-range compression processing to compress the dynamic range of the radiographic image data.

The control section 23 of the console 2 executes the image processing on the radiographic image data, generates image data for the confirmation image to display the confirmation image, and displays the confirmation image on the display unit 21 of the console 2. Accordingly, the operator can confirm the captured radiographic image on the display unit 21. In this description, the image data for the confirmation image are generated after the radiographic image data are image-processed under the set image processing conditions. However, in order to quickly confirm the radiographic image, when receiving the radiographic image data, the control section may first generate the image data for the confirmation image based on the radiographic image data with the set image processing conditions, and then executes the image processing on the radiographic image data under the set image processing conditions.

When completing the receipt of the radiographic image data from the X-ray image detector 1 normally, the control section 23 of the console 2 sends a radiographic image data receipt-completion signal to the X-ray image detector 1 that transmitted the data. When the X-ray image detector 1 receives the radiographic image data receipt-completion signal, the detector control section 17 deletes the radiographic image data already transmitted out of the radiographic image data stored in the image memory 14. Here, the radiographic image data may be transmitted to the console 2 each time of radiography and deleted sequentially after transmitting the image data out of the data stored in the image memory 14. Alternatively, when provided with a large capacity of memory capable of storing data for a plurality of radiographic images, the radiographic image data may be transmitted to the console 2 when all radiography has been finished for one patient 6, or when a certain number of radiography has been finished, and be deleted sequentially after transmission of the image data out of the data stored in the image memory 14.

<Second Embodiment>

The present embodiment is a modification of the first embodiment. The embodiment will now be described hereunder with respect to differences from the first embodiment, and the description of the same parts as of the first embodiment will be omitted.

In the first embodiment, before the X-ray image detector 1 transmits radiographic image data, the detector 1 inquires all consoles 2 for their holding operator ID, but in the embodiment, when the operator ID held by a console 2 changes, the console 2 sends the change of operator relation to all X-ray image detectors. That is, when the control section 23 of the console 2 establishes the operator relation as explained in the first embodiment, the control section controls the communication unit 22 to ask all of the X-ray image detectors 1 to send the information on the operator relation between the console ID and the operator ID, and when the operator relation is cancelled as explained in the first embodiment, the control section controls the communication unit 22 to ask all of the X-ray image detectors 1 to send the operator relation cancel information including the console ID, the cancel information showing the cancellation of the operator relation.

In the X-ray image detector 1, the storage unit 18 of the detector control section 17 stores the operator relation between the console ID and the operator ID, and each time the detector 1 receives from each console 2 the operator relation information or the operator relation cancel information, the control section 17 rewrites the operator relation between the console ID and the operator ID that is stored in the storage unit 18. When transmitting the radiographic image data, the detector 1, using the operator ID relating to the radiographic image data, selects a destination to be sent based on the operator relation stored in the storage unit 18, and transmits the radiographic image data with the radiographic image ID to the selected console 2.

This embodiment is beneficial in the case that the storage unit 18 is consists of a nonvolatile memory and the X-ray image detector 1 is always connected to the network 4. In the first embodiment, on the contrary, even when the detector 1 is not always connected to the network 4 due to turning off the power or shutting down wireless communication, the system is operable when the detector 1 is connected to the network 4 before radiography and before and after the transmission of the radiographic image data, therefore the system has high flexibility.

<Third Embodiment>

The present embodiment is a modification of the first embodiment. The embodiment will now be described hereunder with respect to differences from the first embodiment, and the description of the same parts as of the first embodiment will be omitted.
While the X-ray image detector 1, in the first embodiment, asks each console 2 a held operator ID before transmitting the radiographic image data, the detector 1 in this embodiment transmits to all consoles 2 the radiographic image data together with the radiographic image ID and the operator ID. When the communication unit 22 receives the radiographic image data together with the radiographic image ID and the operator ID, the control section 23 of the console 2 determines whether the received operator ID matches the holding operator ID. When determined that it matches, the control section 23 stores the received radiographic image data into the image memory 25, and, as in the first embodiment, executes image processing and displays the confirmation image on the display unit 21. Thus, only the console 2, in which the matching operator ID is registered, can display the confirmation image on the display unit 21. Here, the transmitted radiographic image data are stored into the image memory 25 only when the operator ID is determined to match, but alternatively, all transmitted radiographic image data may be stored into the image memory 25, and only when the operator ID matches, the control section executes the image processing for obtaining the confirmation image and displays it on the display unit 21. In this alternative, an operator having the privilege of confirming all radiographic images obtained by radiography can be set, and the console 2 relating to the operator ID can display all confirmation images of the radiographic images on the display unit 21.

Meanwhile, the embodiment is useful in a network in which one time transmission of data can be simultaneously received by a plurality of devices. On the contrary, the first embodiment is useful in a network in which device-to-device communication is established for communicating through the network. In the latter case of network, step-by-step communication is necessary for sending to all consoles 2 and fairly large capacity of radiographic image data flow the network repeatedly, thereby causing large network load.

<Fourth Embodiment>

The present embodiment is a modification of the first embodiment. The embodiment will now be described hereunder with respect to differences from the first embodiment, and the description of the same parts as of the first embodiment will be omitted.
While, in the first embodiment, each console 2 holds the operator ID, but in the present embodiment, a server 3 holds the operator relation between a console 2 and the operator ID. When the operator ID held by the console 2 changes, the console 2 sends the change of operator relation to the server 3. That is, when the control section 23 of the console 2 establishes the operator relation as explained in the first embodiment, the control section controls the communication unit 22 to ask the server 3 to send the information on the operator relation between the console ID and the operator ID, and when the operator relation is cancelled as explained in the first embodiment, the control section controls the communication unit 22 to ask the server 3 to send the operator relation cancel information including the console ID, the cancel information showing the cancellation of the operator relation.

The server 3 functions as a relation establishing means for establishing the relation between the console 2 and the operator ID of the operator currently using the console 2, based on the operator relation information and the operator relation cancel information received from each console 2, using the operator ID obtained by the operator ID input unit 26 of the console 2.

Each console 2, in the first embodiment, holds the image relation among the radiographic image ID, the radiographic image detector ID obtained by the detector ID obtaining means of the console 2 and the operator ID, but in the embodiment, all of the image relation obtained by each console 2 are centralized to the server 3 and held therein. That is, when the control section 23 of the console 2 establishes the image relation as explained in the first embodiment, the control section controls the communication unit 22 to send to the server 3 the information on the image relation between the console 1 and the operator ID.

The server 3 functions as the image relation establishing means for establishing the image relation of whole system, based on the image relation among the radiographic image ID, the radiographic image detector ID obtained by the detector ID obtaining means of the console 2 and the operator ID, which was sent from each console 2.

When the X-ray image detector 1 transmits the radiographic image data, the detector 1 sends to the server 3 a handling-console inquiring signal including the operator ID relating to the radiographic image data. When the server 3 receives the handling-console inquiring signal, the server 3 sends to the X-ray image detector 1 a handling-console reply signal including the console ID relating to the received operator ID. That is, responding to the inquiry from the X-ray image detector 1, the server 3 sends the handling-console reply signal including the console ID of the console 2, in which the operator ID relating to the radiographic image ID of the radiographic image is the operator ID of the operator currently using the console, based on the image relation and the operator relation.

Based on the received handling-console reply signal, the X-ray image detector 1 selects the console 2 for transmitting thereto, and transmits the radiographic image data with the radiographic image ID to the selected console 2. When transmitting the radiographic image data, the X-ray image detector 1 sends to the server 3 the handling-console inquiring signal including the operator ID relating to the radiographic image data, and when receiving the handling-console inquiring signal, the server 3 sends to the X-ray image detector 1 the handling-console reply signal including the console ID relating to the received operator ID. Then, based on the received handling-console reply signal, the X-ray image detector 1 selects the console 2 for transmitting thereto, and transmits the radiographic image data with the radiographic image ID to the selected console 2. Thus, the server 3 can collectively manage the operator relation. On the contrary, the first to third embodiments do not need the server 3.

Of course, the invention is not limited to these embodiments, and various variations and modifications may be made appropriately.

The radiography system of the above-described embodiments is particularly useful when the X-ray image detector 1 is a portable FPD cassette, where the radiographic image data of a radiographic image are transmitted to the console 2 in which the matching operator ID is registered, and the confirmation image can be displayed on the display unit. Accordingly, in case that a plurality of radiography rooms are provided, each room having a console 2, and an operator moves room to room carrying the portable FPD cassette with him/her for radiography, the operator can display a confirmation image of the radiographic image transmitted from the X-ray image detector 1 using the console 2 in a radiography room different from the room where the image has been captured, and can immediately confirm the radiographic image data that has been captured by the portable FPD cassette.

In the embodiments described above, the operator relation, detecting conditions and X-ray irradiation control conditions are directly sent from the console 2 to the X-ray irradiation controller 11 and the X-ray image detector 1 through the network 4, but the invention is not limited to this, and these conditions may be once sent to the server 3 through the network 4, and then the server 3 sends them to the X-ray irradiation controller 11 and the X-ray image detector 1.

The X-ray image detector 1 may include a portable memory as a storage unit, which is removably mounted separately from the image memory 14, for storing the radiographic image data that has been detected by the flat surface detector 13, and may be provided with a card slot for mounting it at the side of the detector 1. The portable memory may be a storage medium, such as, for example, a memory card, which may be mounted on the card slot (not shown) provided on the radiographic image detector 3. The portable memory may have a small capacity to store one image, or may have a large capacity to store a plurality of images. Providing a large capacity of memory allows plural times of continuous radiography without transmitting the radiographic image data each time one image is captured.

In the case that the portable memory is provided other than the image memory 14, the image data of the radiographic image detected by flat surface detector 13 may be stored in the image memory 14 or in the portable memory, or may be stored into both of the image memory 14 and the portable memory.

When the radiographic image data is stored in the portable memory, obtaining of the radiographic image data by the console 2 may be performed with the portable memory carried by the operator to the console 2 and mounted on a given mounting position.

On course, the invention is not limited to the embodiments described above.

<Fifth to Eighth Embodiments>

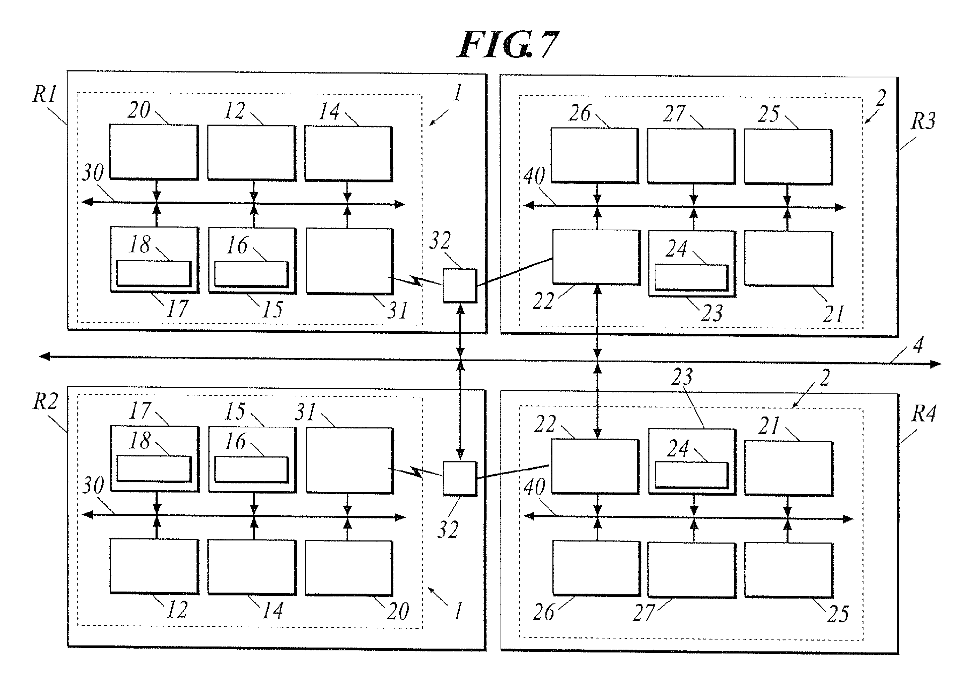

A description will be given of fifth to eighth embodiments of a radiography system according to the invention with reference to FIG. 7. The fifth to eighth embodiments are modifications of the first to fourth embodiments, respectively. The fifth to eighth embodiments will be described hereunder with respect to differences from the first to fourth embodiments, and the description of the same parts as of the first to fourth embodiments will be omitted.

The X-ray radiography system according to the fifth embodiment is, as shown in FIG. 7, a system in which the embodiment is assumed to be applied to X-ray radiography performed within a hospital. There are arranged, for example, X-ray radiography rooms R1 and R2 where X rays are irradiated to a subject, and X-ray control rooms R3 and R4 where an X-ray engineer operates to control X rays irradiating the subject and to perform image processing on the X-ray image obtained by the irradiation of X rays, and the like. FIG. 7 shows a configuration that has two X-ray radiography rooms and two X-ray control rooms, respectively, but the number is not limited to this, and the configuration may have more rooms or have one room, respectively.

Each of the X-ray control rooms R3 and R4 has a console 2 installed therein. The console 2 in the embodiment is capable of sending/receiving various kinds of information to/from X-ray image detectors to be explained later and other external devices. The console 2 controls the entire X-ray radiography system so as to control X-ray radiography and perform image processing on the obtained X-ray image.

The console 2, as in the first embodiment, includes operator ID input unit 26, input operation unit 27, image memory 25, display unit 21, and communication unit 22. The control section 23 including the storage unit 24 controls operation of each unit of the console 2.

The communication unit 22 is connected, for example, to an X-ray source, not shown, and a wireless relay 32 to be explained later, respectively, through the network 4, so as to communicate with the X-ray image detector 1 through the wireless relay 32. That is, the communication unit 22 can send control signals based on instructed content to the X-ray source 4 and the X-ray image detector 1 through the wireless relay 32 by a wireless method using analogue communication or digital communication, and can also receive by the wireless method various kinds of information, such as signals of transmitting operation status and image data from the cassette, and the like.

In the above configuration, respective consoles 2 are installed in the X-ray control rooms R3 and R4, but the console 2 may be a portable terminal capable of wireless communication. In this case, it is preferable that wireless relays are also installed in the X-ray control rooms R3 and R4 and the communication unit 22 can wireless-communicate with the wireless relays 32 within the radiography rooms R1 and R2 and also within the control rooms R3 and R4 to thereby communicate with the X-ray image detector 1 both in the radiography rooms R1 and R2 and the control rooms R3 and R4. This allows the operator to confirm the X-ray image and start image processing for the X-ray image data by the console 2 while giving instructions of radiography positions and the like to the operator not only in the control rooms R3 and R4 as in the past but also in the radiography rooms R1 and R2. Thus, the operator can confirm the X-ray image and start image processing for the X-ray image data during the time for moving between the radiography rooms R1 and R2 and the control rooms R3 and R4. This allows improvement of total radiography efficiency of entire X-ray radiography process that requires repetition of a cycle from X-ray radiography to confirmation of the X-ray image.

There are installed in each of the X-ray radiography rooms R1 and R2 an X-ray source (not shown) for irradiating X rays to a subject, an X-ray image detector 1 for detecting X rays irradiated to the subject to obtain image data, and a wireless relay 32 for relaying the communication between the X-ray image detector 1 and the console 2.

In the embodiment, the X-ray image detector 1 has a housing (not shown) made of lightweight metal like aluminum, magnesium, etc, the housing protecting the inside to make the detector portable. The use of lightweight metal for the housing makes the housing maintain the strength. The operator adjusts the position of the X-ray image detector 1 before X-ray radiography so that X rays can transmit through the subject at the desired position. This adjustment allows the X rays transmitted through the subject irradiated from the X-ray source 4 to be incident on the X-ray image detector 1. The structure of the X-ray image detector 1 is not limited to that exemplified above.

The X-ray image detector 1 is, similar to the first embodiment, provided with, for example, detector control section 17 having storage unit 18, flat surface detector 12, communication unit 31, image memory 14, power supply 15 having battery 16, and display unit 20. Detector control section 17 having storage unit 18, flat surface detector 12, communication unit 31, image memory 14, power supply 15, and display unit 20 are respectively connected to a bus 30 within the X-ray image detector 1.

The communication unit 31 constitutes a detector communication means as in the first embodiment, and is connected to the network 4 through the wireless relay 32. The communication unit 31 is a wireless communication unit capable of sending/receiving various signals by wireless communication with external devices, such as consoles, through the wireless relay 32 and the network 4, and can transmit X-ray image data to any one of the plurality of consoles 2.

The wireless relay 32 communicates with the X-ray image detector 1 with a wireless communication method as described above. The wireless relay 32 is connected to the network 4 through, for example, a communication cable, and can communicate with a plurality of consoles 2 and other external devices that are connected to the network 4. The X-ray image detector 1 receives various signals sent from the console 2 through the wireless relay 32, and also sends various signals to the console 2. Thus, by arranging the wireless relays 32, which are connected to the network 4 with cables, in the X-ray radiography rooms R1 and R2, even when the X-ray image detectors 1 are used in the radiography rooms R1 and R2 that are separated from the external devices such as consoles 2 by a radiation shielding member, good wireless communication can be achieved between the X-ray image detector 1 and the external device such as the console 2.

Here, the communication cable detachably connects the wireless relay 32 to the network 4.

In the embodiment, the wireless relay 32 may have a function of a battery charger that charges the battery 16 of the power source 15 in the X-ray image detector 1. In this case, the wireless relay 32 is provided with a connector, not shown, to charge the battery 16 when the connector is connected to the X-ray image detector 1. The wireless relay 32 is preferably constructed so that attaching/detaching to/from the X-ray image detector 1 can be performed easily. Further, the wireless relay 32 may function as a holder of the X-ray image detector 1 when it is not used, in addition to the function of a battery charger of the detector 1.

Other structure is the same as of the first embodiment, and the description is omitted with the same reference numerals designated for the same components.

Next, a process of the embodiment will be described.
The storage unit 24 of the control section 23 stores radiography programs similar to that of the first embodiment, and the control section 23 establishes the operator relation between the console 2 and the operator ID of the operator currently using the console 2 as in the first embodiment. The control section 23 identifies an X-ray image detector 1 that is to output the radiographic image data due to the radiography, and sends the operator ID, the radiographic image ID, detecting conditions, etc. to the communication unit 31 of the detector 1 through the network 4 and the wireless relay 32. After completion of the radiography, the flat surface detector 12 of the detector 1 detects the radiation based on the detecting conditions and outputs the radiographic image data. In order to confirm the radiographic image on the console 2 having the operator ID relating to the radiographic image data, the detector control section 17 transmits the radiographic image data with the radiographic image ID to the corresponding console 2 from the communication unit 31 through the wireless relay 32 and the network 4.

As described above, according to the embodiment, since the X-ray image detectors 1 communicates with the external device such as the console 2 through the wireless relays 32 with the wireless relays 32 provided in the X-ray radiography rooms R1 and R2, even when the X-ray image detectors 1 are used in the radiography rooms R1 and R2 that are separated from the external devices such as consoles 2 by a radiation shielding member, good wireless communication can be attained between the X-ray image detector 1 and the external device such as the console 2. Further, even when a microwave or light, which have high straight-forwardness and directivity, are used for communication, communication through the wireless relays 32 enables good wireless communication without a communication defect. This permits effective transmission of radiographic images with large capacity at higher speed, thereby more improving radiography efficiency.

Alternatively, the wireless relays 32, the communication unit 22 of the console 2 and the like may be connected to a network through a hub (not shown). A server, not shown, managing a whole system may be connected to the network 4.

Next, a sixth embodiment is shown in FIG. 7 as a whole system, similar to the fifth embodiment, and as in the second embodiment, when the operator ID held by the console 2 changes, the change of the operator relation is sent to all X-ray image detectors 1. That is, when the control section 23 of the console 2 establishes the operator relation as described in the first and fifth embodiments, the control section 23 controls the communication unit 22 to send to all X-ray image detectors 1 the information on the operator relation between the console ID and the operator ID, and when the operator relation is cancelled as described in the first and fifth embodiment, the control section controls the communication unit 22 to send to all X-ray image detectors 1 the operator relation cancel information including the console ID, the information showing the cancellation of the operator relation.
In the X-ray image detector 1, the storage unit 18 of the detector control section 17 stores the operator relation between the console ID and the operator ID, and each time the detector 1 receives from each console 2 the operator relation information or the operator relation cancel information, the control section 17 rewrites the operator relation between the console ID and the operator ID, the relation stored in the storage unit 18. When transmitting the radiographic image data, the detector 1, using the operator ID relating to the radiographic image data, selects a destination to be sent based on the operator relation stored in the storage unit 18, and transmits the radiographic image data with the radiographic image ID to the selected console 2. Other points are the same as of the second embodiment.

A seventh embodiment is shown in FIG. 7 as a whole system, similar to the fifth and sixth embodiments, and as in the third embodiment, the X-ray image detector 1 transmits to all consoles 2 the radiographic image data together with the radiographic image ID and the operator ID. When the communication unit 22 receives the radiographic image data together with the radiographic image ID and the operator ID, the control section 23 of the console 2 determines whether the received operator ID matches the holding operator ID. When determined that it matches, the control section 23 stores the received radiographic image data into the image memory 25, and, as in the first embodiment, executes image processing and displays the confirmation image on the display unit 21. Thus, only the console 2, in which the matching operator ID is registered, can display the confirmation image on the display unit 21. Other points are the same as of the third embodiment.

Further, an eighth embodiment is shown in FIG. 7 as a whole system, similar to the fifth and seventh embodiments. As in the fourth embodiment, the system has a server (not shown) connected to the network 4, and the server holds the operator relation between a console 2 and the operator ID. When the operator ID held by the console 2 changes, the console 2 sends the change of the operator relation to the server. That is, when the control section 23 of the console 2 establishes the operator relation as explained in the first embodiment, the control section controls the communication unit 22 to send to the server 3 the information on the operator relation between the console ID and the operator ID, and when the operator relation is cancelled as explained in the first embodiment, the control section controls the communication unit 22 to send to the server 3 the operator relation cancel information including the console ID, the cancel information showing the cancellation of the operator relation. The server functions as a relation establishing means for establishing the relation between the console 2 and the operator ID of the operator currently using the console 2, based on the operator relation information and the operator relation cancel information received from each console 2, using the operator ID obtained by the operator ID input unit 26 of the console 2.

Of course, the invention is not limited to the first to eighth embodiments, and various variations and modifications may be made appropriately.

The entire disclosure of Japanese Patent Application No. 2004-113549 which was filed on April 7, 2004, including specification, claims, drawings and abstract, is incorporated into a part of the present invention in its entirety.

Industrial Applicability

In the radiography system including a plurality of consoles and one or more radiographic image detectors, even when an operator moves from one console to another, the operator can confirm a radiographic image on a console currently used by the operator and radiography efficiency of the radiography can be improved. Accordingly, the system is applicable to radiography performed at hospitals, image diagnosis center and the like.

Explanation of Reference Numerals

1    radiographic image detector
2    console
3    server
4    network
5    DICOM network
6    patient
12   flat surface detector
17   detector control section
21   display unit
22   communication unit
23   console control section
25   image memory
26   operator ID input unit
27   input operation unit

**Claims**

1.  A radiography system comprising:

    one or more radiographic image detectors, each detector obtaining radiographic image data representing a radiographic image captured by radiography; and
    a plurality of consoles, each console displaying a confirmation image corresponding to the radiographic image with use of the received radiographic image data, when obtaining an operator ID of an operator of the console and receiving the radiographic image data representing the radiographic image from the radiographic image detector,
    wherein a relation between the console and the operator ID of the operator currently using the console is held based on the operator ID obtained by the console, and
    the console in which the operator ID corresponding to the radiographic image is the operator ID of the operator currently using the console, enables displaying an image for confirming the radiographic image thereon.

2.  The radiography system of claim 1, wherein the radiographic image detector holds the relation between the operator ID relating to the radiographic image captured by the radiography and the console, and transmits the radiographic image data representing the radiographic image to the console in which the operator ID relating to the radiographic image is the operator ID of the operator currently using the console.

3.  The radiography system of claim 2, wherein the radiographic image detector transmits, based on the relation, to the console in which the operator ID relating to the captured radiographic image is the operator ID of the operator currently using the console.

4.  The radiography system of claims 2 or 3, wherein the system has the radiographic image detectors, and the plurality of consoles obtain a radiographic image detector ID relating to a radiographic image and hold the image relation among a radiographic image ID, the radiographic image detector ID obtained by the consoles and the operator ID; and the radiographic image detector obtains the relation between the radiographic image ID relating to the radiographic image detector and the operator ID based on the image relation, and transmits the radiographic image data of the radiographic image to the console in which the operator ID corresponding to the radiographic image ID of the radiographic image is the operator ID of the operator currently using the console.

5. The radiography system of claim 1,
wherein the system has the consoles and a server connected to the radiographic image detectors; and the server holds the relation between the console and the operator ID of the operator currently using the console.

6. The radiography system of claim 1, wherein each of the consoles holds an operator ID having the relation with the console whereby the plurality of consoles holds the relation.

7. The radiography system of claim 6,
wherein the radiographic image detector holds the relation between the radiographic image ID and the operator ID, and transmits, by using the relation, the operator ID of the radiographic image to the plurality of consoles; and
the console determines whether the received operator ID of the radiographic image matches the operator ID of the operator currently using the console, the console, when determined it matches, sends an operator ID matching signal to the radiographic image detector, the radiographic image detector, when receiving the operator ID matching signal, transmits the radiographic image data of the radiographic image to the console that sent the operator ID matching signal, and then the console can display the confirmation image corresponding to the radiographic image using the received radiographic image data of the radiographic image.

8. The radiography system of any one of claims 1 to 7, wherein a console already having the relation with one operator ID does not establish relation with other operator ID.

9. The radiography system of any one of claims 1 to 8, wherein, when one operator ID has newly established relation with the one console, the relation having been established with a console other than the one console for the one operator ID is canceled.

10. The radiography system of any one of claims 1 to 9, wherein the console has an operation input unit, and wherein, when a predetermined term during which the operation input unit does not have input thereto meets a given condition, the operator relation of the console is canceled.

11. The radiography system of any one of claims 1 to 10, wherein the operator relation of the console is canceled when the operator operates the console.

12. The radiography system of any one of claims 1 to 11, wherein at least one of the radiographic image detectors is a portable type cassette FPD.

13. The radiography system of any one of claims 1 to 12, wherein the radiographic image detector communicates by wireless communication.

14. The radiography system of claim 13, wherein the wireless communication is one using a microwave or light.

15. The radiography system of claims 13 or 14, further comprising a wireless relay for relaying the wireless communication.

16. The radiography system of claim 15, further comprising a plurality of radiography rooms, each room being provided with the radiographic image detector and the wireless relay, wherein the plurality of wireless relays are connected to the plurality of consoles through a network.

17. A radiography program for causing a computer of a console in a radiography system, to carry out functions,
wherein the system comprises:

one or more radiographic image detectors, each detector obtaining radiographic image data representing a radiographic image captured by radiography, based on an operator ID of an operator currently using the console, the ID received from the console, holding relation between the operator ID relating to the radiographic image captured by the radiography and the console, and transmitting, based on the relation, the radiographic image data of the radiographic image to the console relating to the operator ID that corresponds to the captured radiographic image; and
the plurality of consoles, each console including an operator ID obtaining unit for obtaining the operator ID of the operator, a communication unit for communicating with the radiographic image detector, a display unit for displaying a confirmation image, and the computer, and

the program causes the computer to carry out the functions as:

a relation transmission control section to control the communication unit so as to send to the radiographic image detector the operator ID of the operator currently using the console based on the operator ID obtained by the operator ID obtaining unit; and a display control section to make the display unit display the confirmation image corresponding to the radiographic image by using the radiographic image data received according to the operational input to the operation input unit when the communication unit receives the radiographic image data representing the radiographic image relating to the operator ID of the operator currently using the console.

18. A radiography program for causing a computer of a console in a radiography system, to carry out functions,
wherein the system comprises:

one or more radiographic image detectors, each detector obtaining radiographic image data representing a radiographic image captured by radiography, inquiring a server for a console to which the radiographic image data of the radiographic image are to be transmitted, and transmitting the radiographic image data of the radiographic image to the console resulted from the inquiring;
the server for communicating with one or more radiographic image detectors and a plurality of consoles, holding relation between the console and an operator ID of an operator currently using the console, and responding to the inquiry from the radiographic image detector based on the relation; and
the plurality of consoles, each console including an operation input unit to be operated by the operator, an operator ID obtaining unit for obtaining the operator ID of the operator, a communication unit for communicating with the server and one or more radiographic image detectors, a display unit for displaying a confirmation image, and a computer, and
the program causes the computer to carry out the functions as:

a relation transmission control section to control the communication unit so as to send to the server the operator ID of the operator currently using the console based on the operator ID obtained by the operator ID obtaining unit; and

a display control section to make the display unit display the confirmation image corresponding to the radiographic image by using the radiographic image data received according to the operational input to the operation input unit when the communication unit receives the radiographic image data representing the radiographic image relating to the operator ID of the operator currently using the console.

19. A radiography program for causing a computer of a console in a radiography system, to carry out functions,
wherein the system comprises:

a plurality of radiographic image detectors, each detector obtaining radiographic image data representing a radiographic image captured by radiography, obtaining an operator ID relating to the radiographic image, transmitting the obtained operator ID relating to the radiographic image to all of a plurality of consoles, receiving an operator ID matching signal from a console, and transmitting the radiographic image data of the radiographic image to the console that sent the operator ID matching signal; and
the plurality of consoles, each console including an operation input unit to be operated by the operator, an operator ID obtaining unit for obtaining the operator ID of the operator, a communication unit for communicating with the plurality of radiographic image detectors, a display unit for displaying a confirmation image, and a computer, and
the program causes the computer to carry out the functions as:

an operator ID holding section to hold the operator ID of the operator currently using the console based on the operator ID obtained by the operator ID obtaining unit;
an operator ID matching determination section to determine whether the received operator ID matches the operator ID of the operator currently using the console when the communication unit receives the operator ID from the radiographic image detector;
an operator ID matching signal communication section to send the operator ID matching signal to the radiographic image detector when the operator ID matching determination section determines it matches; and
a display control section to make the display unit display the confirmation image relating to the radiographic image by using the radiographic image data received according to the operational input to the operation input unit.

20. A radiography program for causing a computer of a console in a radiography system, to carry out functions,
wherein the system comprises:

a plurality of radiographic image detectors, each detector obtaining radiographic image data representing a radiographic image captured by radiography, obtaining an operator ID relating to the radiographic image, transmitting the radiographic image data of the captured radiographic image and the operator ID to all of a plurality of consoles; and
the plurality of consoles, each console including an operation input unit to be operated by the operator, an operator ID obtaining unit for obtaining the operator ID of the operator, a communication unit for communicating with the plurality of radiographic image detectors, a display unit for displaying a confirmation image, and a computer, and
the program causes the computer to carry out the functions as:

an operator ID holding section to hold the operator ID of the operator currently using the console based on the operator ID obtained by the operator ID obtaining unit; and
a display control section to make the display unit display the confirmation image relating to the radiographic image by using the received radiographic image data according to the operational input to the operation input unit when the communication unit receives from the radiographic image detector the radiographic image data representing the radiographic image and the operator ID, and the received operator ID matches the operator ID of the operator currently using the console.

# FIG.1

# FIG.2

RADIATION

# FIG.3

# FIG.4

# FIG.5

START

INPUT OPERATOR ID — S1

OPERATOR RELATION EXISTS? — S2

YES

NO — S3

ESTABLISH RELATION BETWEEN OPERATOR ID AND CONSOLE

DISPLAY THAT OTHER OPERATOR USES THIS CONSOLE — S4

END

# FIG.6

START COUNTING TIME
FROM INPUT OPERATION — *S11*

PREDETERMINED
TIME ELAPSED? — *S12*

NO

YES

CANCEL RELATION
BETWEEN OPERATOR ID
AND CONSOLE — *S13*

# FIG.7

EP 1 733 681 A1

<table>
<tr><td colspan="2" align="center">INTERNATIONAL SEARCH REPORT</td><td>International application No.<br>PCT/JP2005/006852</td></tr>
</table>

A. CLASSIFICATION OF SUBJECT MATTER
Int.Cl$^7$ A61B6/00, A61B5/00, G01T1/00, G03B42/02, G01T1/20, G01T1/24

According to International Patent Classification (IPC) or to both national classification and IPC

B. FIELDS SEARCHED

Minimum documentation searched (classification system followed by classification symbols)
Int.Cl$^7$ A61B6/00-6/14, A61B5/00, G03B42/02, G01T1/00-1/40

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2003-24319 A (Hitachi Medical Corp.),<br>28 January, 2003 (28.01.03),<br>Full text; Figs. 1 to 2<br>(Family: none) | 1-20 |
| A | JP 2003-220035 A (Hitachi Medical Corp.),<br>05 August, 2003 (05.08.03),<br>Full text; Figs. 1 to 2<br>(Family: none) | 1-20 |
| A | JP 2002-136512 A (Toshiba Corp.),<br>14 May, 2002 (14.05.02),<br>Full text; Figs. 1 to 21<br>& WO 200236014 A1     & EP 1336378 A1<br>& US 2003191389 A1 | 1-20 |

| | | |
|---|---|---|
| ☒ Further documents are listed in the continuation of Box C. | ☐ See patent family annex. | |

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>28 April, 2005 (28.04.05) | Date of mailing of the international search report<br>24 May, 2005 (24.05.05) |
|---|---|
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

International application No.

PCT/JP2005/006852

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2002-363 A  (Daifuku Co., Ltd.), 08 January, 2002 (08.01.02), Full text; Figs. 1 to 20 (Family: none) | 1-20 |

Form PCT/ISA/210 (continuation of second sheet) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002159476 A
- JP 2004180931 A
- JP 2004173907 A
- JP 2004113549 A